# FASCICULE DE BREVET EUROPEEN

(11) **EP 1 087 976 B1**
(45) Date de publication et mention de la délivrance du brevet: **14.08.2002**
(21) Numéro de dépôt: 99923711.8
(22) Date de dépôt: 10.06.1999
(51) Int. Cl.: C07D 495/04, A61K 31/44

(54) **FORME POLYMORPHE DE L'HYDROGENOSULFATE DE CLOPIDOGREL**
POLYMORPHE FORM VON CLOPIDOGREL-HYDROGENOSULFAT
POLYMORPHIC CLOPIDOGREL HYDROGENESULPHATE FORM

(30) Priorité: 15.06.1998 FR 9807464
(43) Date de publication de la demande: 04.04.2001
(73) Titulaire: SANOFI-SYNTHELABO, 75013 Paris (FR)
(72) Inventeur: BOUSQUET, André, F-04200 Sisteron (FR); CASTRO, Bertrand, F-94270 Kremlin-Bicêtre (FR); SAINT-GERMAIN, Jean, F-04200 Sisteron (FR)
(74) Mandataire: Thouret-Lemaitre, Elisabeth
(86) Numéro de dépôt international: FR9901371
(87) Numéro de publication internationale: WO9965915

(56) Documents cités:
- EP-A- 0 281 459
- EP-A- 0 465 358

## Description

La présente invention conceme un nouveau polymorphe de l'hydrogénosulfate de clopidogrel ou hydrogénosulfate de (+)-(S)-α-(2-chlorophényl)-4,5,6,7-tétrahydrothiéno [3,2-c]pyridinyl-5-acétate de méthyle et un procédé pour sa préparation. Plus particulièrement, l'invention se rapporte à la préparation de ce polymorphe appelé Forme 2 et à l'isolement de ce composé sous cette nouvelle forme cristalline, ainsi qu'aux compositions pharmaceutiques la contenant.

L'hydrogénosulfate de clopidogrel est un antithrombotique qui a été décrit pour la première fois dans EP 281459. Le procédé de synthèse revendiqué dans ce brevet permet la préparation de l'hydrogénosulfate de clopidogrel que l'on appellera Forme 1.

Il a maintenant été découvert que l'hydrogénosulfate de clopidogrel peut exister sous différentes formes cristallines polymorphes qui diffèrent les unes des autres par leur stabilité, par leurs propriétés physiques, par leurs caractéristiques spectrales et par leur procédé de préparation.

Ainsi une de ces nouvelles formes polymorphes fait l'objet de la présente invention, elle est décrite dans la présente demande et sera nommée Forme 2.

La présente invention concerne également un procédé pour la préparation de l'hydrogénosulfate de clopidogrel sous sa forme polymorphe 2.

Le brevet EP 281459 décrit des énantiomères de dérivés de tétrahydrothiénopyridines et de leurs sels pharmaceutiquement acceptables. EP 281459 revendique spécifiquement l'hydrogénosulfate de clopidogrel, c'est-à-dire l'isomère dextrogyre, qui possède une excellente activité antiagrégante plaquettaire alors que l'isomère lévogyre est moins actif et moins bien toléré.

Le brevet EP 281459 déposé il y a dix ans ne fait aucune référence à l'existence de formes polymorphiques spécifiques de l'hydrogénosulfate de clopidogrel. La synthèse décrite dans EP 281459 permet la préparation l'hydrogénosulfate du polymorphe du clopidogrel Forme 1. EP 281459 ne suggère pas non plus l'existence de différentes formes polymorphes du clopidogrel ou de l'hydrogénosulfate de clopidogrel.

Selon tous les enseignements des documents ci-dessus, l'isomère dextrogyre du clopidogrel est préparé par salification du composé racémique par un acide optiquement actif comme l'acide 10-L-camphosulfonique dans l'acétone suivie de recristallisations successives du sel jusqu'à l'obtention d'un produit à pouvoir rotatoire constant, puis libération de l'isomère dextrogyre de son sel par une base. L'hydrogénosulfate de clopidogrel est ensuite obtenu de façon classique par dissolution de la dite base dans l'acétone refroidie dans la glace et ajout d'acide sulfurique concentré jusqu'à précipitation. Le précipité ainsi obtenu est alors isolé par filtration, lavé et séché pour fournir l'hydrogénosulfate de clopidogrel sous forme de cristaux blancs dont le point de fusion est 184°C et le pouvoir rotatoire +55,1° (c = 1,891 / CH₃OH).

Les procédés de synthèse décrits dans l'art antérieur permettent uniquement la synthèse de l'hydrogénosulfate de clopidogrel Forme 1.

Ainsi la présente invention concerne la forme polymorphe nommée Forme 2 de l'hydrogénosulfate de clopidogrel, qui comme la Forme 1 de ce composé est utile en tant que médicament pour la prophylaxie et le traitement de la thrombose en agissant comme antiagrégant plaquettaire. En ce qui concerne l'utilisation du clopidogrel et de ses sels, on pourra se référer à Drugs of the Future 1993, 18, 2, 107-112. La Forme 2 polymorphe de l'hydrogénosulfate de clopidogrel est donc utilisée en tant que principe actif pour la préparation d'un médicament, en association avec au moins un excipient pharmaceutiquement acceptable, dans les mêmes indications que la Forme 1.

Il a été maintenant trouvé que si l'on cristallise l'hydrogénosulfate de clopidogrel dans un solvant, on peut obtenir soit la forme cristalline correspondant à celle du produit obtenu selon EP 281459 cités ci-dessus, Forme 1, soit une forme cristalline nouvelle, très stable, ayant une structure bien définie, ci-après désignée Forme 2. Plus particulièrement, il a été trouvé que la nouvelle forme cristalline de l'hydrogénosulfate de clopidogrel, Forme 2 est au moins aussi stable que la Forme 1 décrite et qu'elle ne se transforme pas spontanément dans la Forme 1 précédemment connue. De plus, la poudre obtenue à partir de la Forme 2 est plus compacte et beaucoup moins électrostatique que celle obtenue à partir de la Forme 1 et peut donc être plus aisément soumise à tout traitement dans les conditions usuelles de technique pharmaceutique et en particulier de galénique industrielle.

Il a par ailleurs été constaté que la Forme 2 présente une plus faible solubilité que la Forme 1 résultant de sa plus grande stabilité thermodynamique.

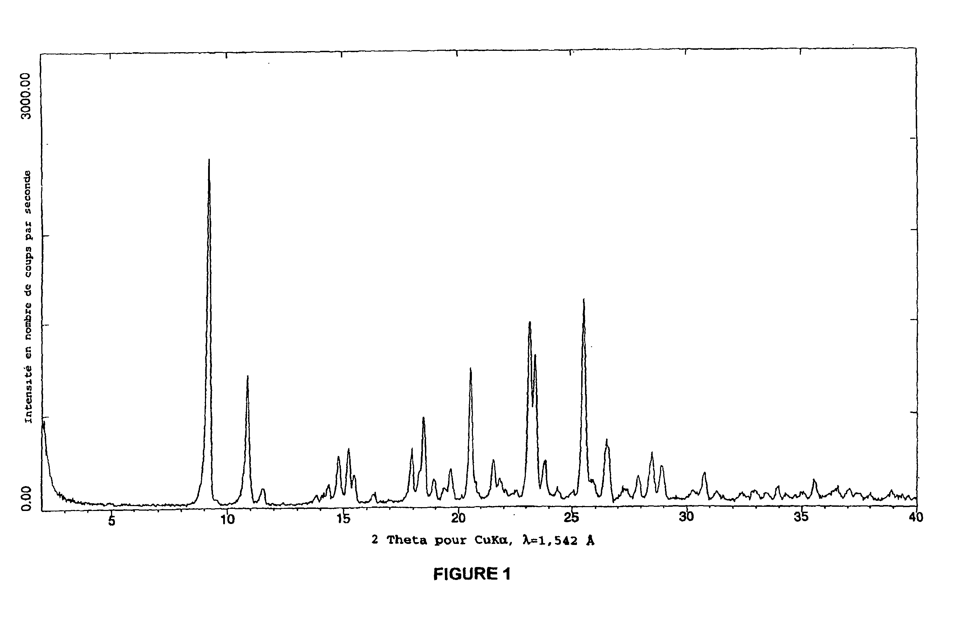

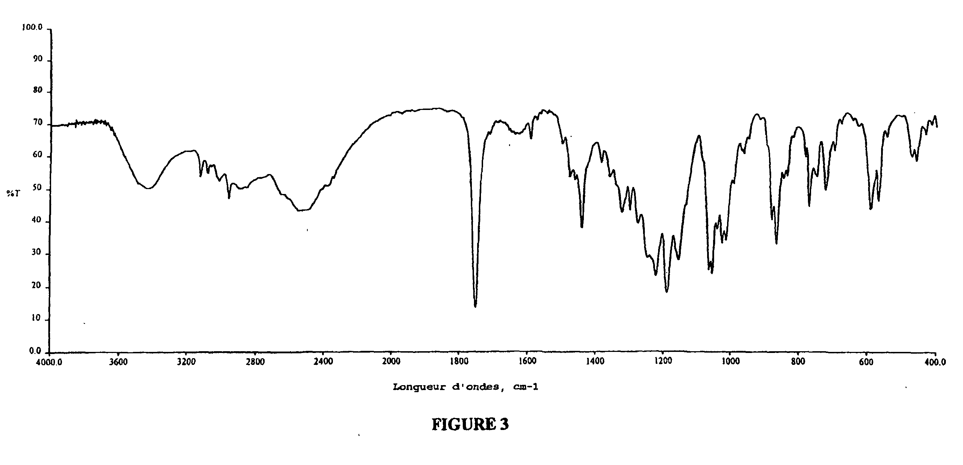

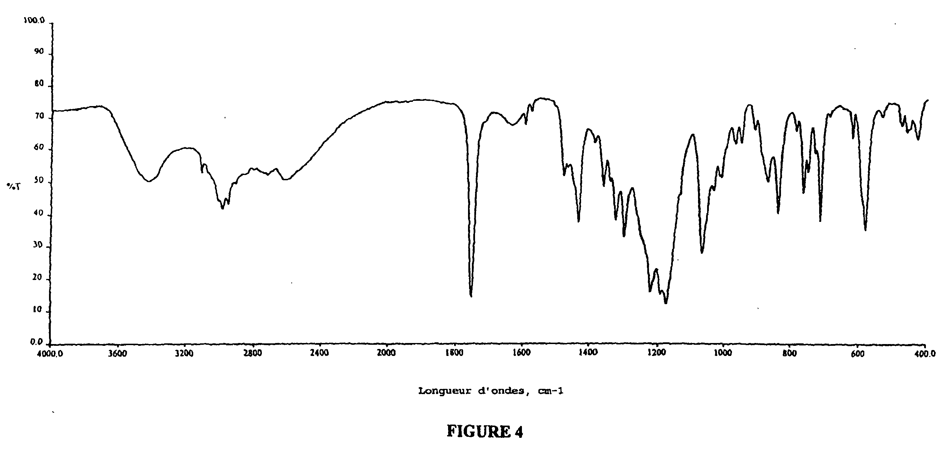

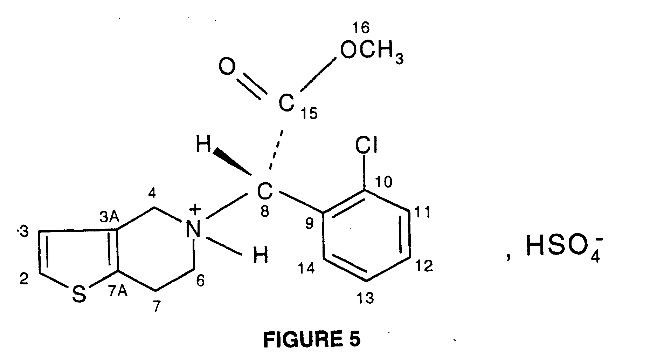

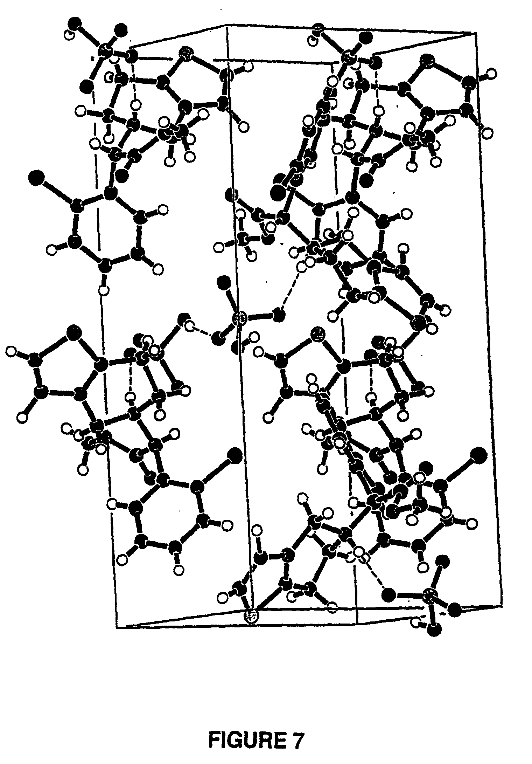

La différence entre la nouvelle forme cristalline de l'hydrogénosulfate de clopidogrel selon la présente invention, Forme 2 et la Forme 1, ressort de l'examen des Figures 1 à 4, alors que les Figures 5 à 7 font état de la structure dans les cristaux de la Forme 2.

Les figures 1 à 7 sont caractérisées comme suit :
- la **Figure 1** donne le diffractogramme de poudre des rayons X de l'hydrogénosulfate de clopidogrel Forme 1 ;
- la **Figure 2** montre le diffractogramme de poudre des rayons X de l'hydrogénosulfate de clopidogrel Forme 2 ;
- la **Figure 3** montre le spectre infrarouge de la Forme 2 ;
- la **Figure 4** montre le spectre infrarouge de la Forme 1 ;
- la **Figure 5** montre la formule développée de l'hydrogénosulfate de clopidogrel avec la numérotation des atomes sous la Forme cristalline 2 ;
- la **Figure 6** montre la conformation spatiale de l'hydrogénosulfate de clopidogrel Forme 2
- la **Figure 7** montre l'empilement des molécules de l'hydrogénosulfate de clopidogrel Forme 2 dans la maille du cristal.

Il a été constaté d'après les données cristallographiques, que la structure cristalline de la Forme 1 contient deux cations libres dans le cristal du clopidogrel et deux anions bisulfate libres. Les deux cations libres sont de conformation similaire.

Selon les données cristallographiques de la Forme 2, il a été constaté qu'elle contient un cation libre dans le cristal - anion bisulfate pair.

Dans les deux formes, les cations sont protonés de manière axiale et l'atome d'azote est de configuration R ; la conformation des cations dans la Forme 2 est différente de celle observée dans la Forme 1.

Dans l'arrangement moléculaire des deux formes cristallines, aucun site n'est occupé par des molécules de solvant.

L'arrangement des anions est très différent de l'une à l'autre des deux structures cristallines. La structure cristalline de la Forme 2, de type orthorhombique, est moins dense (1,462 g / cm³) que la structure cristalline de la Forme 1, de type monoclinique, (1,505 g / cm³).

Selon un autre de ses aspects, la présente invention a pour objet un procédé pour la préparation de la Forme 2 de l'hydrogénosulfate de clopidogrel caractérisé en ce que :
(a) on met en solution du camphosulfonate de (+)-(S)-α-(2-chlorophényl)-4,5,6,7-tétrahydrothiéno[3,2-c]pyridinyl-5-acétate de méthyle dans un solvant organique,
(b) on extrait l'acide camphosulfonique par une solution aqueuse alcaline de carbonate de potassium et on lave à l'eau,
(c) on concentre la phase organique sous vide et on reprend le résidu de concentration dans l'acétone,
(d) on ajoute de l'acide sulfurique à 80%,
(e) on chauffe à reflux, le produit cristallise, on refroidit, on filtre et on lave les cristaux qui sont ensuite séchés sous pression réduite, pour fournir l'hydrogénosulfate de clopidogrel Forme 1,
(f) les eaux-mères hydroacétoniques résultantes, finissent par relarguer au bout de 3 à 6 mois des cristaux d'hydrogénosulfate de clopidogrel Forme 2.

Ainsi, la présente invention concerne un procédé pour la préparation du (+)-(S) hydrogénosulfate de clopidogrel Forme 2, caractérisé en ce que :
les eaux-mères hydroacétoniques résultantes de la cristallisation de la Forme 1 du (+)-(S) hydrogénosulfate de clopidogrel finissent par relarguer au bout de 3 à 6 mois des cristaux d'hydrogénosulfate de clopidogrel Forme 2.

Les eaux-mères hydroacétoniques résultantes de la cristallisation de la Forme 1 du (+)-(S) hydrogénosulfate de clopidogrel contiennent de 0,3 à 1 % d'eau.

Elles contiennent jusqu'à environ 10 % d'hydrogénosulfate de clopidogrel, cette quantité étant calculée à partir de la quantité du camphosulfonate (+)-(S)-α-(2-chlorophényl)-4,5,6,7-tétrahydrothiéno[3,2-c]pyridinyl-5-acétate de méthyle utilisée au cours de la transformation en hydrogénosulfate.

Ces eaux-mères hydroacétoniques relarguent lentement, au bout d'une durée de trois à six mois, à une température inférieure à 40°C, l'hydrogénosulfate de clopidogrel Forme 2.

Selon un autre de ses aspects, la présente invention concerne un autre procédé pour la préparation de la Forme 2 de l'hydrogénosulfate du clopidogrel caractérisé en ce que :
(a) on met en solution du camphosulfonate de (+)-(S)-a-(2-chlorophényl)-4,5,6,7-tétrahydrothiéno[3,2-c]pyridinyl-5-acétate de méthyle dans un solvant organique,
(b) on extrait l'acide camphosulfonique par une solution aqueuse alcaline de carbonate de potassium et on lave à l'eau,
(c) on concentre la phase organique sous vide et on reprend le résidu de concentration dans l'acétone,
(d) on ajoute de l'acide sulfurique à 96 % à 20°C et on amorce avec de l'hydrogénosulfate de clopidogrel Forme 2,
(e) le produit cristallise, on refroidit, on filtre et on lave les cristaux qui sont ensuite séchés sous pression réduite, pour fournir l'hydrogénosulfate de clopidogrel Forme 2.

Une autre alternative consiste à soumettre la suspension cristalline à un cisaillement mécanique à l'aide d'un dispositif de cisaillement. Ce dispositif peut atteindre une vitesse de rotation d'environ 10 000 à 15 000 tours par minutes. Des dispositifs pourvus de ces caractéristiques sont par exemple de type Turrax® commercialisés par IKA-Werke (DE). Ces dispositifs sont par ailleurs adaptés au traitement de quantités industrielles.

Le principe est d'obtenir par broyage de fines particules à partir d'une solution de base ne contenant qu'une fraction de l'acide sulfurique total. La portion restante sera alors coulée lentement pour favoriser la croissance cristalline. Des essais ont été réalisés en partant de 10% de l'acide sulfurique nécessaire versé au départ.

Ainsi, la présente invention a pour objet la Forme 2 de l'hydrogénosuffate de clopidogrel caractérisée par le profil de diffraction des rayons X de la poudre donné dans le TABLEAU I.

Plus particulièrement, la Forme 2 est aussi caractérisée par un point de fusion, déterminé par analyse enthalpique différentielle (DSC), de 176°C et par des absorptions caractéristiques dans l'infrarouge et dans le proche infrarouge.

Certaines propriétés physiques et le comportement de la nouvelle forme cristalline de l'hydrogénosulfate de clopidogrel selon la présente invention sont complètement différentes de ceux de la Forme 1 comme il a été démontré en examinant les deux formes selon les méthodes et les techniques classiques.

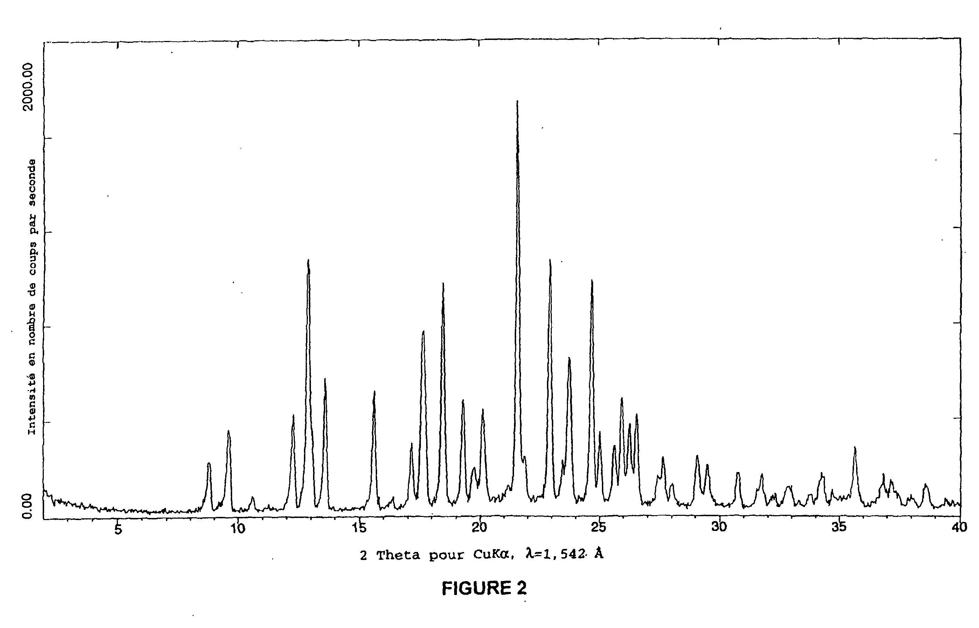

Le profil de diffraction des rayons X de la poudre (angle de diffraction) a été établi avec un diffractomètre Siemens D500TT. Les diffractogrammes de poudre caractéristiques entre 2 et 40° en 2θ de Bragg (2 théta, deg., pour CuKα, λ=1,542 Å) sont présentés sur la Figure 1 pour la Forme 1 et sur la Figure 2 pour la Forme 2. Les raies significatives de la Figure 1 sont consignées dans le TABLEAU II, alors que celles de la Figure 2 sont rassemblées dans le TABLEAU I.

Dans les TABLEAUX I et II, d est la distance interréticulaire et I/I₀ représente l'intensité relative, exprimée en pourcentage de la raie la plus intense.

**TABLEAU I :**

| Forme 2 Raies significatives de la Figure 2. | |
|---|---|
| **d(Å)** | **I/I**_{**0**} |
| 4,11 | 100,0 |
| 6,86 | 61,7 |
| 3,87 | 61,4 |
| 3,60 | 56,3 |
| 4,80 | 55,8 |
| 5,01 | 44,4 |
| 3,74 | 37,9 |
| 6,49 | 33,1 |
| 5,66 | 29,8 |

**TABLEAU II :**

| Forme 1 Raies significatives de la Figure 1 | |
|---|---|
| **d(Å)** | **I/I**_{**0**} |
| 9,60 | 100,0 |
| 3,49 | 58,8 |
| 3,83 | 52,0 |
| 3,80 | 42,5 |
| 4,31 | 39,0 |
| 8,13 | 37,2 |
| 4,80 | 25,5 |
| 3,86 | 19,1 |
| 5,80 | 16,8 |
| 4,95 | 16,8 |

L'analyse enthalpique différentielle (DSC) des Formes 1 et 2 a été réalisée comparativement en utilisant un appareil DSC7 Perkin Elmer, calibré par référence à l'indium. Pour l'analyse calorimétrique on a utilisé 2,899 mg de la Forme 1 ou 2,574 mg de la Forme 2, telle qu'obtenue à l'EXEMPLE 2, dans une coupelle en aluminium sertie et percée, dans une zone de température de 40 à 230°C à une vitesse de chauffage de 10°C/minute. Le point de fusion et l'enthalpie de fusion sont indiqués dans le TABLEAU III. Le point de fusion correspond à la température caractéristique de la fusion obtenue par DSC. Cette valeur peut être définie également comme étant la température correspondant à l'intersection entre la ligne de base et la tangente à la montée du pic de fusions observées par DSC.

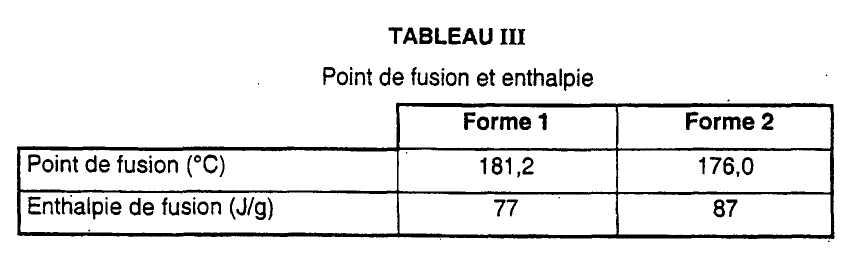

La différence entre la nouvelle Forme 2 et la Forme 1 de l'hydrogénosulfate de clopidogrel a été également mise en évidence par spectroscopie infrarouge. Les spectres IR à Transformée de Fourier (FTIR) ont été obtenus avec un spectromètre Perkin Elmer système 2000 de résolution 4 cm⁻¹ de 4000 cm⁻¹ à 400 cm⁻¹. Les échantillons se présentent sous forme de pastille de KBr à 0,3 % en Forme 1 ou en Forme 2. La pastille a été comprimée sous 10 tonnes pendant 2 minutes. Chaque échantillon a été examiné après 4 accumulations.
La comparaison de raies caractéristiques, en termes de longueur d'onde (en cm⁻¹) et d'intensité (en pourcentage de transmittance) est illustrée dans le TABLEAU IV.

**TABLEAU IV**

| Spectre infra-rouge | | | |
|---|---|---|---|
| **Forme 1** | | **Forme 2** | |
| Longueur d'onde (cm⁻¹) | % de transmittance | Longueur d'onde (cm⁻¹) | % de transmittance |
| 2987 | 42 | 2551 | 43 |
| 1753 | 14 | 1753 | 13,4 |
| 1222 | 16 | 1497 | 63,7 |
| 1175 | 12 | 1189 | 18 |
| 841 | 40 | 1029 | 33,2 |

Il ressort du TABLEAU IV que la Forme 2 présente des absorptions caractéristiques à 2551 cm⁻¹, 1497 cm⁻¹, 1189 cm⁻¹ et 1029 cm⁻¹ qui sont absentes de la Forme 1.

La structure particulière de la poudre de la Forme 2 a été mise en évidence par une analyse du mono-cristal par diffraction des rayons X de la poudre en utilisant un diffractomètre MSC-Rigaka AFC6S et les logiciels SHELXS-90 et SHELXS-93 sur une station de travail SG IRIS Indigo. La position des hydrogènes C-H a été générée à une distance de 0,95Å. Les données cristallographiques, notamment les distances interplanaires (a,b,c) les angles (α,β,γ) et le volume de chaque cellule unitaire, sont indiqués dans le TABLEAU V.

**TABLEAU V**

| Données cristallographiques et établissement de la structure de la Forme 2 | |
|---|---|
| **Système cristallin Groupe spatial** | **Orthorhombique P2**_{**1**}**2**_{**1**}**2**_{**1**} |
| Dimensions de la cellule unitaire : | |
| a | 10,321 (6) Å |
| b | 20,118 (9) Å |
| c | 9,187 (7) Å |
| α | 90 degrés |
| β | 90 degrés |
| γ | 90 degrés |
| volume | 1908 (2) Å³ |
| Z | 4 |
| densité (calculée) | 1,462 g/cm³ |
| réflexions collectées | 2134 |
| facteur R | 0,0473 |

Les coordonnées atomiques de la Forme 2 sont données dans le TABLEAU VI, la longueur des liaisons dans le TABLEAU VII, les angles entre les liaisons dans le TABLEAU VIII et les angles de torsion caractéristiques dans le TABLEAU IX.

**TABLEAU VI**

| Paramètres de position de la Forme 2 | | | | |
|---|---|---|---|---|
| **atome** | **x** | **y** | **z** | **U(eq)** |
| Cl(1) | 0,2223(3) | 0,21728(12) | 0,4295(3) | 0,0835(8) |
| S(1) | 0,8085(2) | -0,00068(11) | 0,3557(3) | 0,0724(7) |
| S(2) | 0,2840(2) | 0,01908(8) | 0,0013(2) | 0,0412(4) |
| O(1) | 0,3030(7) | 0,2376(3) | -0,0528(7) | 0,087(2) |
| O(2) | 0,4630(6) | 0,1637(3) | -0,0860(6) | 0,060(2) |
| O(3) | 0,2175(6) | -0,0350(3) | 0,0957(6) | 0,0551(14) |
| O(4) | 0,2728(6) | -0,0093(3) | -0,1432(5) | 0,074(2) |
| O(5) | 0,4174(4) | 0,0241(2) | 0,0497(6) | 0,0503(13) |
| O(6) | 0,2146(5) | 0,0800(2) | 0,0199(7) | 0,065(2) |
| N(5) | 0,4936(6) | 0,1343(3) | 0,1946(7) | 0,0380(14) |
| C(2) | 0,9111(10) | 0,0427(5) | 0,2500(13) | 0,081(3) |
| C(3A) | 0,7214(7) | 0,1002(3) | 0,2215(9) | 0,047(2) |
| C(3) | 0,8554(8) | 0,0950(5) | 0,1824(11) | 0,060(2) |
| C(4) | 0,6332(7) | 0,1548(4) | 0,1706(10) | 0,044(2) |
| C(6) | 0,4750(8) | 0,1100(4) | 0,3487(9) | 0,045(2) |
| C(7) | 0,5487(8) | 0,0450(4) | 0,3722(10) | 0,051(2) |
| C(7A) | 0,6833(8) | 0,0526(3) | 0,3144(9) | 0,050(2) |
| C(8) | 0,3940(8) | 0,1880(4) | 0,1574(9) | 0,043(2) |
| C(9) | 0,4119(7) | 0,2523(3) | 0,2360(9) | 0,044(2) |
| C(10) | 0,3435(8) | 0,2688(4) | 0,3613(10) | 0,057(2) |
| C(11) | 0,3630(10) | 0,3292(4) | 0,4290(11) | 0,076(3) |
| C(12) | 0,4545(10) | 0,3734(4) | 0,3773(12) | 0,080(3) |
| C(13) | 0,5223(10) | 0,3579(4) | 0,2550(12) | 0,067(3) |
| C(14) | 0,5019(8) | 0,2980(3) | 0,1863(10) | 0,052(2) |
| C(15) | 0,3823(8) | 0,1995(4) | -0,0079(11) | 0,053(2) |
| C(16) | 0,4462(16) | 0,1687(6) | -0,2422(11) | 0,096(4) |

**TABLEAU VII**

| Distances intramoléculaires dans la Forme 2 | | |
|---|---|---|
| **atome** | **atome** | **distance** |
| CI(1) | C(10) | 1,742(8) |
| S(1) | C(2) | 1,682(12) |
| S(1) | C(7A) | 1,722(8) |
| S(2) | O(6) | 1,429(5) |
| S(2) | O(4) | 1,450(5) |
| S(2) | O(5) | 1,450(5) |
| S(2) | O(3) | 1,551(5) |
| O(1) | C(15) | 1,195(9) |
| 0(2) | C(15) | 1,314(10) |
| O(2) | C(16) | 1,448(10) |
| N(5) | C(6) | 1,510(10) |
| N(5) | C(4) | 1,515(9) |
| N(5) | C(8) | 1,530(9) |
| C(2) | C(3) | 1,350(13) |
| C(3A) | C(7A) | 1,341(10) |
| C(3A) | C(3) | 1,432(10) |
| C(3A) | C(4) | 1,501(10) |
| C(6) | C(7) | 1,528(10) |
| C(7) | C(7A) | 1,495(11) |
| C(8) | C(9) | 1,493(10) |
| C(8) | C(15) | 1,541(12) |
| C(9) | C(14) | 1,384(10) |
| C(9) | C(10) | 1,390(11) |
| C(10) | C(11) | 1,379(11) |
| C(11) | C(12) | 1,382(12) |
| C(12) | C(13) | 1,359(13) |
| C(13) | C(14) | 1,378(11) |

Les distances sont en Ångstroms. Les écart types estimés sur la décimale sont entre parenthèses.

**TABLEAU VIII**

| Angles entre les liaisons intramoléculaires impliquant des atomes non hydrogène | | | |
|---|---|---|---|
| **atome** | **atome** | **atome** | **angle** |
| C(2) | S(1) | C(7A) | 91,2(4) |
| O(6) | S(2) | O(4) | 114,0(4) |
| O(6) | S(2) | O(5) | 112,3(3) |
| O(4) | S(2) | O(5) | 112,6(3) |
| O(6) | S(2) | O(3) | 108,2(3) |
| O(4) | S(2) | O(3) | 101,6(3) |
| O(5) | S(2) | O(3) | 107,3(3) |
| C(15) | O(2) | C(16) | 115,3(9) |
| C(6) | N(5) | C(4) | 110,1(6) |
| C(6) | N(5) | C(8) | 110,6(6) |
| C(4) | N(5) | C(8) | 114,5(5) |
| C(3) | C(2) | S(1) | 113,7(7) |
| C(7A) | C(3A) | C(3) | 113,0(8) |
| C(7A) | C(3A) | C(4) | 122,8(7) |
| C(3) | C(3A) | C(4) | 124,1(8) |
| C(2) | C(3) | C(3A) | 110,7(9) |
| C(3A) | C(4) | N(5) | 109,5(6) |
| N(5) | C(6) | C(7) | 110,2(7) |
| C(7A) | C(7) | C(6) | 108,9(6) |
| C(3A) | C(7A) | C(7) | 124,9(7) |
| C(3A) | C(7A) | S(1) | 111,4(6) |
| C(7) | C(7A) | S(1) | 123,7(6) |
| C(9) | C(8) | N(5) | 114,9(6) |
| C(9) | C(8) | C(15) | 110,9(6) |
| N(5) | C(8) | C(15) | 112,2(7) |
| C(14) | C(9) | C(10) | 117,1(7) |
| C(14) | C(9) | C(8) | 119,9(8) |
| C(10) | C(9) | C(8) | 123,0(7) |
| C(11) | C(10) | C(9) | 120,7(8) |
| C(11) | C(10) | Cl(1) | 117,8(7) |
| C(9) | C(10) | Cl(1) | 121,4(6) |
| C(10) | C(11) | C(12) | 120,7(9) |
| C(13) | C(12) | C(11) | 119,3(9) |
| C(12) | C(13) | C(14) | 120,0(9) |
| C(13) | C(14) | C(9) | 122,2(9) |
| O(1) | C(15) | O(2) | 126,7(9) |
| O(1) | C(15) | C(8) | 119,3(9) |
| O(2) | C(15) | C(8) | 114,0(7) |

Les angles sont en degrés. Les écarts types estimés sur la dernière décimale sont entre parenthèses.

**TABLEAU IX**

| Angles de conformation et torsion caractéristique | | | | |
|---|---|---|---|---|
| **(1)** | **(2)** | **(3)** | **(4)** | **angle** |
| C(7A) | S(1) | C(2) | C(3) | -1,1(9) |
| S(1) | C(2) | C(3) | C(3A) | 0,9(12) |
| C(7A) | C(3A) | C(3) | C(2) | 0,0(12) |
| C(4) | C(3A) | C(3) | C(2) | 177,1(8) |
| C(7A) | C(3A) | C(4) | N(5) | -19,7(11) |
| C(3) | C(3A) | C(4) | N(5) | 163,4(8) |
| C(6) | N(5) | C(4) | C(3A) | 50,2(8) |
| C(8) | N(5) | C(4) | C(3A) | 175,7(7) |
| C(4) | N(5) | C(6) | C(7) | -67,3(8) |
| C(8) | N(5) | C(6) | C(7) | 165,0(6) |
| N(5) | C(6) | C(7) | C(7A) | 47,8(9) |
| C(3) | C(3A) | C(7A) | C(7) | -179,1(8) |
| C(4) | C(3A) | C(7A) | C(7) | 3,8(13) |
| C(3) | C(3A) | C(7A) | S(1) | -0,8(9) |
| C(4) | C(3A) | C(7A) | S(1) | -177,9(6) |
| C(6) | C(7) | C(7A) | C(3A) | -17,6(12) |
| C(6) | C(7) | C(7A) | S(1) | 164,3(6) |
| C(2) | S(1) | C(7A) | C(3A) | 1,1(7) |
| C(2) | S(1) | C(7A) | C(7) | 179,4(8) |
| C(6) | N(5) | C(8) | C(9) | 68,9(8) |
| C(4) | N(5) | C(8) | C(9) | -56,3(10) |
| C(6) | N(5) | C(8) | C(15) | -163,2(6) |
| C(4) | N(5) | C(8) | C(15) | 71,6(8) |
| N(5) | C(8) | C(9) | C(14) | 81,4(9) |
| C(15) | C(8) | C(9) | C(14) | -47,2(10) |
| N(5) | C(8) | C(9) | C(10) | -97,3(9) |
| C(15) | C(8) | C(9) | C(10) | 134,2(8) |
| C(14) | C(9) | C(10) | C(11) | 1,9(12) |
| C(8) | C(9) | C(10) | C(11) | -179,4(8) |
| C(14) | C(9) | C(10) | Cl(1) | 176,9(6) |
| C(8) | C(9) | C(10) | Cl(1) | -4,4(11) |
| C(9) | C(10) | C(11) | C(12) | -2,6(14) |
| Cl(1) | C(10) | C(11) | C(12) | -177,8(8) |
| C(10) | C(11) | C(12) | C(13) | 3(2) |
| C(11) | C(12) | C(13) | C(14) | -2(2) |
| C(12) | C(13) | C(14) | C(9) | 1,1(14) |
| C(10) | C(9) | C(14) | C(13) | -1,1(12) |
| C(8) | C(9) | C(14) | C(13) | -179,9(8) |
| C(16) | O(2) | C(15) | O(1) | -4,3(13) |
| C(16) | O(2) | C(15) | C(8) | 174,5(8) |
| C(9) | C(8) | C(15) | O(1) | -54,0(10) |
| N(5) | C(8) | C(15) | O(1) | 176,0(7) |
| C(9) | C(8) | C(15) | O(2) | 127,1(7) |
| N(5) | C(8) | C(15) | O(2) | -2,8(9) |

Les angles sont en degrés. Les écarts type estimés sur la dernière décimale sont entre parenthèses.

Le signe est positif si, en regardant de l'atome 2 à l'atome 3, par un mouvement dans le sens horaire l'atome 1 se superpose sur l'atome 4.

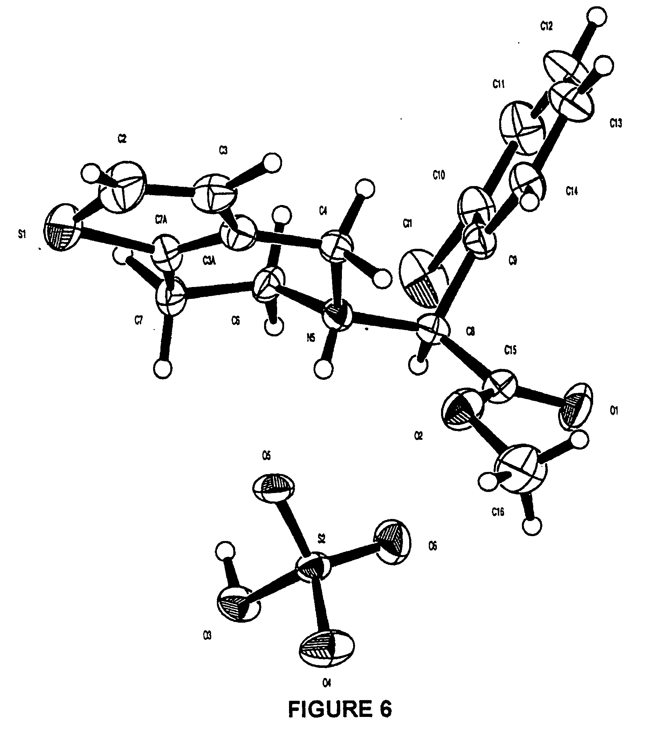

L'étude cristallographique aux rayons X, notamment les données cristallographiques du TABLEAU I, les coordonnées atomiques du TABLEAU VI, la longueur des liaisons du TABLEAU VII, les angles entre les liaisons du TABLEAU VIII et les angles de torsion caractéristiques du TABLEAU IX prouvent la structure proposée et illustrée sur les Figures 5 et 6.

L'examen au microscope a révélé que les cristaux de la nouvelle Forme 2 sont morphologiquement différents de ceux de la Forme 1.

Les cristaux de la Forme 1 se présentent sous forme de plaques irrégulières, tandis que les cristaux de la Forme 2 se présentent sous forme d'agglomérats.

Grâce à sa faible électrostaticité par rapport à celle de la Forme 1, elle est donc particulièrement adaptée à la fabrication de compositions pharmaceutiques pour le traitement de toutes les maladies dans lesquelles un antithrombotique est indiqué.

Ainsi, selon un autre de ses aspects, la présente invention a pour objet des compositions pharmaceutiques contenant comme principe actif, la Forme 2 de l'hydrogénosulfate de clopidogrel caractérisée par le profil de diffraction des rayons X de la poudre illustré dans le TABLEAU I.

De préférence, la Forme 2 de l'hydrogénosulfate de clopidogrel selon la présente invention est formulée dans des compositions pharmaceutiques par voie orale contenant 75 mg de principe actif par unité de dosage, en mélange avec au moins un excipient pharmaceutique.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Si on souhaite formuler le principe actif pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables.

Le principe actif peut être formulé également sous forme de microcapsules, éventuellement avec un ou plusieurs supports ou additifs.

Les EXEMPLES suivants illustrent l'invention sans toutefois la limiter.

### Préparation du camphosulfonate de (+)-(S)-α-(2-chlorophényl)-4,5,6,7-tétrahydrothiéno [3,2-c]pyridinyl-5-acétate de méthyle.

Dans un réacteur agité, on charge 400 kg de chlorhydrate de l'α-(2-chlorophényl)-4,5,6,7-tétrahydrothiéno[3,2-c]pyridinyl-5-acétate de méthyle racémique et 1840 kg de dichlorométhane. On ajoute ensuite lentement 1200 kg d'une solution aqueuse de bicarbonate de sodium à 8%. Après décantation, la phase organique est concentrée sous vide. Le résidu de concentration est dilué avec 1000 litres d'acétone. On ajoute à 20-25°C une solution de 154 kg d'acide 1 R-10 camphosulfonique dans 620 litres d'acétone. On refroidit et cristallise le camphosulfonate de l'α-(2-chlorophényl)-4,5,6,7-tétrahydrothiéno[3,2-c]pyridinyl-5-acétate de méthyle en amorçant si necessaire. Lorsque la cristallisation est abondante, on chauffe à reflux puis on refroidit à 25°C. Les cristaux sont ensuite filtrés et lavés à l'acétone puis séchés sous pression réduite. On obtient ainsi 196 kg de camphosulfonate de (+)-(S)-α-(2-chlorophényl)-4,5,6,7-tétrahydrothiéno[3,2-c]pyridinyl-5-acétate de méthyle soit un rendement de 33%.

### Préparation de l'hydrogénosulfate de clopidogrel Forme 2

### EXEMPLE 1 A

Dans un réacteur de 250 ml, sous azote, on introduit 50 g de camphosulfonate de clopidogrel préparé comme indiqué ci-dessus. On ajoute 100 ml de dichlorométhane et le mélange réactionnel est agité pendant 10 minutes. Puis on introduit une solution de 9,1 g de carbonate de potassium en solution dans 70 ml d'eau désionisée. On soutire la phase organique et lave plusieurs fois la phase aqueuse au dichlorométhane. On rassemble les phases organiques et les concentre sous vide. On ajoute 229 ml d'acétone sur le concentrat et on filtre sur fritté de 0,1 µ à 0,22 µ. On charge la solution acétonique contenant la base dans un réacteur sous azote et on additionne alors 7,4 g d'une solution d'acide sulfurique à 80 %, à 20°C, puis on chauffe le mélange jusqu'au reflux ; la cristallisation débute et on maintient le reflux pendant 2 heures.

On distille le solvant, refroidi à une température de 0 à -5°C et sépare les cristaux par filtration sur Büchner pour obtenir après séchage 21,4 g de la Forme 2 d'hydrogénosulfate de clopidogrel ; F = 176 ± 3°C.

### EXEMPLE 1 B

Dans un réacteur de 6000 litres, sous azote, on introduit 1200 kg de camphosulfonate de clopidogrel préparé comme indiqué ci-dessus. On ajoute 2345 litres de dichlorométhane et le mélange réactionnel est agité pendant 30 minutes à 1 heure. Puis on introduit une solution de 214,5 kg de carbonate de potassium en solution dans 1827 litres d'eau désionisée. On soutire la phase organique et lave plusieurs fois la phase aqueuse au dichlorométhane. On rassemble les phases organiques et les concentre sous vide. On ajoute de l'acétone sur le concentrat et on filtre sur filtre à cartouche de 0,1 µ à 1 µ. On charge la solution acétonique (3033 litres) contenant la base dans un réacteur sous azote et on additionne alors 264,8 kg d'une solution d'acide sulfurique à 80 % , à 20°C.

On distille le solvant, refroidi à une température de 0 à -5°C et sépare les cristaux par filtration sur Büchner pour obtenir après séchage 779,1 kg de la Forme 1 d'hydrogénosulfate de clopidogrel ; F = 184 ± 3°C.

Les eaux-mères hydroacétoniques résultantes à une température inférieure à 40°C, finissent par relarguer au bout de 3 à 6 mois des cristaux d'hydrogénosulfate de clopidogrel Forme 2 ; F = 176 ± 3°C.

### EXEMPLE 1 C

Dans un réacteur de 6000 litres, sous azote, on introduit 1200 kg de camphosulfonate de clopidogrel préparé comme indiqué ci-dessus. On ajoute 2345 litres de dichlorométhane et le mélange réactionnei est agité pendant 30 minutes à 1 heure. Puis on introduit une solution de 214,5 kg de carbonate de potassium en solution dans 1827 litres d'eau désionisée. On soutire la phase organique et lave plusieurs fois la phase aqueuse au dichlorométhane. On rassemble les phases organiques et les concentre sous vide. On ajoute de l'acétone sur le concentrat et on filtre sur filtre à cartouche de 0,1 µ à 1 µ. On charge la solution acétonique (3033 litres) contenant la base dans un réacteur sous azote et on additionne alors 264,8 kg d'une solution d'acide sulfurique à 96 %, à 20°C.

On distille le solvant, refroidi à une température de 0 à -5°C et sépare les cristaux par filtration sur Büchner pour obtenir après séchage 785,3 kg de la Forme 1 d'hydrogénosulfate de clopidogrel ; F = 184 ± 3°C.

Les eaux-mères hydroacétoniques résultantes à une température inférieure à 40°C, finissent par relarguer au bout de 3 à 6 mois des cristaux d'hydrogénosulfate de clopidogrel Forme 2 ; F = 176 ± 3°C.

### EXEMPLE 2

Dans un réacteur on charge 909 litres de dichlorométhane et 450 kg de camphosulfonate de (+)-(S)-α-(2-chlorophényl)-4,5,6,7-tétrahydrothiéno[3,2-c]pyridinyl-5-acétate de méthyle. On extrait l'acide camphosulfonique par une solution aqueuse de 80 kg de carbonate de potassium dans 680 litres d'eau. La phase organique est ensuite lavée à l'eau. Le dichlorométhane est concentré et le résidu de concentration est repris par 1140 litres d'acétone. On ajoute alors à 20°C, 100 kg d'acide sulfurique à 96 %. On amorce avec 0,3 kg de l'hydrogénosulfate de clopidogrel Forme 2 obtenu selon l'EXEMPLE 1B ou 1C. L'hydrogénosulfate de clopidogrel cristallise. On filtre puis lavé à l'acétone et on sèche sous pression réduite. On obtient 310 kg d'hydrogénosulfate de clopidogrel Forme 2 soit un rendement de 90,9 % ; F = 176 ± 3°C.

### EXEMPLE 3

Dans un réacteur on charge 909 litres de dichlorométhane et 450 kg de camphosulfonate de (+)-(S)-α-(2-chlorophényl)-4,5,6,7-tétrahydrothiéno[3,2-c]pyridinyl-5-acétate de méthyle. On extrait l'acide camphosulfonique par une solution aqueuse de 80 kg de carbonate de potassium dans 680 litres d'eau. La phase organique est ensuite lavée à l'eau. Le dichlorométhane est concentré et le résidu de concentration est repris par 1296 litres d'acétone.

La température est stabilisée à 20°C et le Turrax® est mis en fonctionnement. On ajoute alors 10 % de la quantité d'acide sulfurique à 94-96 % (8,3 kg) en quelques minutes. On amorce avec 0,012 kg d'hydrogénosulfonate de clopidogrel Forme 2 obtenu selon l'EXEMPLE 1B ou1C. L'hydrogénosulfonate de clopidogrel cristallise. On laisse le mélange réactionnel sous l'action du Turrax® pendant 45 minutes. On fait couler alors les 90 % d'acide sulfurique à 94-96 % restant (74,6 kg) en environ 2 heures, tout en continuant à faire fonctionner le Turrax®. On arrête le Turrax® 30 mn après la fin d'ajout d'acide et on agite pendant 30 minutes à 20°C. On filtre, lave à l'acétone et on sèche sous pression réduite.

On obtient 310 kg d'hydrogénosulfonate de clopidogrel Forme 2, soit un rendement de 90,9 % ; F = 176 ± 3°C

## Revendications

1. Polymorphe cristallin (+)-(S) de l'hydrogénosulfate de clopidogrel (Forme 2) dont le diffractogramme de poudre des rayons X montre les pics caractéristiques suivants exprimés en distances interplanaires à approximativement 4,11 ; 6,86 ; 3,60 ; 5,01 ; 3,74 ; 6,49 ; 5,66 Å.

2. Polymorphe cristallin (+)-(S) de l'hydrogénosulfate de clopidogrel (Forme 2) dont le spectre infrarouge présente des absorptions caractéristiques exprimées en cm⁻¹ à : 2551, 1497, 1189 et 1029, avec des pourcentages de transmittance respectifs d'environ : 43 ; 63,7 ; 18 ; 33,2

3. Polymorphe cristallin (+)-(S) de l'hydrogénosulfate de clopidogrel (Forme 2) ayant un point de fusion de 176 ± 3°C.

4. Polymorphe cristallin de l'hydrogénosulfate de clopidogrel (Forme 2) **caractérisé par** le diffractogramme de poudre des rayons X selon la figure 2.

5. Polymorphe cristallin de l'hydrogénosulfate de clopidogrel (Forme 2) **caractérisé par** un spectre infrarouge selon la figure 3.

6. Polymorphe cristallin de l'hydrogénosulfate de clopidogrel (Forme 2) **caractérisé par** le diffractogramme de poudre des rayons X selon la revendication 1 et un spectre infrarouge selon la revendication 2.

7. Procédé pour la préparation du (+)-(S) hydrogénosulfate de clopidogrel Forme 2, selon les revendications 1, 2 et 3, **caractérisé en ce que** :
l'on relargue les eaux-mères hydroacétoniques résultantes de la cristallisation de la Forme 1 du (+)-(S) hydrogénosulfate de clopidogrel pour obtenir au bout de 3 à 6 mois des cristaux d'hydrogénosulfate de clopidogrel Forme 2.

8. Procédé selon la revendication 7 **caractérisé en ce que** les eaux-mères hydroacétoniques résultantes de la cristallisation de la Forme 1 du (+)-(S) hydrogénosulfate de clopidogrel contiennent de 0,3 à 1 % d'eau.

9. Procédé selon la revendication 7 **caractérisé en ce que** les eaux-mères hydroacétoniques résultantes de la cristallisation de la Forme 1 du (+)-(S) hydrogénosulfate de clopidogrel contiennent jusqu'à environ 10 % d'hydrogénosulfate de clopidogrel, cette quantité étant calculée à partir de la quantité du camphosulfonate (+)-(S)-α-(2-chlorophényl)-4,5,6,7-tétrahydrothiéno [3,2-c]pyridinyl-5-acétate de méthyle utilisée au cours de la transformation en hydrogénosulfate.

10. Procédé selon l'une quelconque des revendications 7 à 9 **caractérisé en ce que** les eaux-mères hydroacétoniques résultantes de la cristallisation de la Forme 1 du (+)-(S) hydrogénosulfate de clopidogrel relarguent lentement, au bout d'une durée de trois à six mois, à une température inférieure à 40°C, l'hydrogénosulfate de clopidogrel Forme 2.

11. Procédé pour la préparation de l'hydrogénosulfate du clopidogrel Forme 2 dans lequel :
(a) on met en solution du camphosulfonate de (+)-(S)-a-(2-chlorophényl)-4,5,6,7-tétrahydrothiéno[3,2-c]pyridinyl-5-acétate de méthyle dans un solvant organique,
(b) on extrait l'acide camphosulfonique par une solution aqueuse alcaline de carbonate de potassium et on lave à l'eau,
(c) on concentre la phase organique sous pression réduite et on reprend le résidu de concentration dans l'acétone,
**caractérisé en ce que** on ajoute de l'acide sulfurique à 94-96 % et on amorce avec de l'hydrogénosulfate de clopidogrel Forme 2, on cristallise le produit, on refroidit, on filtre et on lave les cristaux qui sont ensuite séchés sous pression réduite, pour fournir l'hydrogénosulfate de clopidogrel Forme 2.

12. Composition pharmaceutique contenant en tant que principe actif, le polymorphe Forme 2 de l'hydrogénosulfate de clopidogrel selon la revendication 1 en association avec au moins un excipient pharmaceutique.

## Patentansprüche

1. Kristallines (+)-(S)-Polymorph von Clopidogrel-Hydrogensulfat (Form 2), dessen Pulver-Röntgenstrahlenbeugungsdiagramm die folgenden charakteristischen Peaks ausgedrückt als Gitterebenenabstände von etwa 4,11; 6,86; 3,60; 5,01; 3,74; 6,49; 5,66 Å aufweist.

2. Kristallines (+)-(S)-Polymorph von Clopidogrel-Hydrogesulfat (Form 2), dessen Infrarotspektrum charakteristische Absorptionen ausgedrückt in cm⁻¹ bei: 2551, 1497, 1189 und 1029 mit den entsprechenden Transmissionsprozentsätzen von etwa 43; 63,7; 18; 33,2 aufweist.

3. Kristallines (+)-(S)-Polymorph von Clopidogrel-Hydrogesulfat (Form 2) mit einem Schmelzpunkt von 176 ± 3°C.

4. Kristallines Polymorph von Clopidogrel-Hydrogesulfat (Form 2), **gekennzeichnet durch** das Pulver-Röntgenstrahlenbeugungsdiagramm gemäß Figur 2.

5. Kristallines Polymorph von Clopidogrel-Hydrogesulfat (Form 2), **gekennzeichnet durch** das Infrarotspektrum gemäß Figur 3.

6. Kristallines Polymorph von Clopidogrel-Hydrogesulfat (Form 2), **gekennzeichnet durch** das Pulver-Röntgenstrahlenbeugungsdiagramm nach Anspruch 1 und ein Infrarotspektrum nach Anspruch 2.

7. Verfahren zur Herstellung des (+)-(S)-Clopidogrel-Hydrogesulfats (Form 2) nach den Ansprüchen 1, 2 und 3, **dadurch gekennzeichnet, daß** man die bei der Kristallisation der Form 1 von kristallines (+)-(S)-Clopidogrel-Hydrogesulfat anfallenden wäßrig-acetonischen Mutterlaugen aussalzt, so daß man nach Ablauf von 3 bis 6 Monaten Kristalle von Clopidogrel-Hydrogensulfat Form 2 erhält.

8. Verfahren nach Anspruch 7, **dadurch gekennzeichnet, daß** die bei der Kristallisation der Form 1 von (+)-(S)-Clopidogrel-Hydrogesulfat anfallenden wäßrig-acetonischen Mutterlaugen 0,3 bis 1 % Wasser enthalten.

9. Verfahren nach Anspruch 7, **dadurch gekennzeichnet, daß** die bei der Kristallisation der Form 1 von (+)-(S)-Clopidogrel-Hydrogesulfat anfallenden wäßrig-acetonischen Mutterlaugen bis zu etwa 10 % Clopidogrel-Hydrogensulfat enthalten, wobei diese Menge berechnet ist ausgehend von der Menge des im Verlaufe der Umwandlung in das Hydrogensulfat eingesetzten Camphersulfats von (+)-(S)-α-(2-Chlorphenyl)-4,5,6,7-tetrahydrothieno]3,2-c]pyridinyl-5-essigsäuremethylester.

10. Verfahren nach einem der Ansprüche 7 bis 9, **dadurch gekennzeichnet, daß** die bei der Kristallisation der Form 1 des (+)-(S)-Clopidogrel-Hydrogensulfats anfallenden wäßrig-acetonischen Mutterlaugen langsam nach Ablauf einer Dauer von drei bis sechs Monaten bei einer Temperatur unterhalb 40°C das Clopidogrel-Hydrogensulfat Form 2 durch Aussalzen ergeben.

11. Verfahren zur Herstellung von Clopidogrel-Hydrogensulfat Form 2, worin man:
(a) das Camphersulfonat von (+)-(S)-α-(2-Chlorphenyl)-4,5,6,7-tetrahydrothieno[3,2-clpyridinyl-5-essigsäuremethylester in einem organischen Lösungsmittel löst,
(b) die Camphersulfonsäure mit einer wäßrigen alkalischen Kaliumcarbonatlösung extrahiert und mit Wasser wäscht,
(c) die organische Phase unter vermindertem Druck einengt und den Konzentrationsrückstand in Aceton aufnimmt,
**dadurch gekennzeichnet, daß** man 94-96 %-ige Schwefelsäure zugibt und die Kristallisation mit Clopidogrel-Hydrogensulfat Form 2 auslöst, das Produkt kristallisiert, abkühlt, filtriert und die Kristalle wäscht, welche anschließend unter vermindertem Druck getrocknet werden unter Bildung von Clopidogrel-Hydrogensulfat Form 2.

12. Pharmazeutische Zubereitung enthaltend als Wirkstoff das Polymorph Form 2 von Clopidogrel-Hydrogensulfat nach Anspruch 1 in Kombination mit mindestens einem pharmazeutischen Trägermaterial.

## Claims

1. Crystalline (+)-(S) polymorph of clopidogrel hydrogen sulphate (Form 2) whose powder X-ray diffractogram shows the following characteristic peaks expressed as interplanar distances at approximately 4.11; 6.86; 3.60; 5.01; 3.74; 6.49; 5.66 Å.

2. Crystalline (+)-(S) polymorph of clopidogrel hydrogen sulphate (Form 2) whose infrared spectrum exhibits characteristic absorptions expressed in cm⁻¹ at: 2551, 1497, 1189 and 1029, with respective percentages of transmittance of about: 43; 63.7; 18; 33.2.

3. Crystalline (+)-(S) polymorph of clopidogrel hydrogen sulphate (Form 2) having a melting point of 176 ± 3°C.

4. Crystalline polymorph of clopidogrel hydrogen sulphate (Form 2) **characterized by** the powder X-ray diffractogram according to Figure 2.

5. Crystalline polymorph of clopidogrel hydrogen sulphate (Form 2) **characterized by** an infrared spectrum according to Figure 3.

6. Crystalline polymorph of clopidogrel hydrogen sulphate (Form 2) **characterized by** the powder X-ray diffractogram according to Claim 1 and an infrared spectrum according to Claim 2.

7. Method for the preparation of (+)-(S)-clopidogrel hydrogen sulphate Form 2, according to Claims 1, 2 and 3, **characterized in that**:
the aqueous-acetone mother liquors resulting from the crystallization of (+)-(S)-clopidogrel hydrogen sulphate Form 1 release, after 3 to 6 months, crystals of clopidogrel hydrogen sulphate Form 2.

8. Method according to Claim 7, **characterized in that** the aqueous-acetone mother liquors resulting from the crystallization of (+)-(S)-clopidogrel hydrogen sulphate Form 1 contain 0.3 to 1% of water.

9. Method according to Claim 7, **characterized in that** the aqueous-acetone mother liquors resulting from the crystallization of (+)-(S)-clopidogrel hydrogen sulphate Form 1 contain up to about 10% of clopidogrel hydrogen sulphate, this quantity being calculated from the quantity of methyl (+)-(S)-α-(2-chlorophenyl)-4,5,6,7-tetrahydrothieno[3,2-c]pyridinyl-5-acetate camphorsulphonate used during the conversion to hydrogen sulphate.

10. Method according to any one of Claims 7 to 9,
**characterized in that** the aqueous-acetone mother liquors resulting from the crystallization of (+)-(S)-clopidogrel hydrogen sulphate Form 1 release slowly, after a period of three to six months, at a temperature of less than 40°C, clopidogrel hydrogen sulphate Form 2.

11. Method for the preparation of clopidogrel hydrogen sulphate Form 2 in which:
(a) methyl (+)-(S)-α-(2-chlorophenyl)-4,5,6,7-tetrahydrothieno[3,2-c]pyridinyl-5-acetate camphorsulphonate is dissolved in an organic solvent,
(b) camphorsulphonic acid is extracted with an aqueous alkaline solution of potassium carbonate and washed with water,
(c) the organic phase is concentrated under reduced pressure and the concentration residue is taken up in acetone,
**characterized in that** 94-96% sulphuric acid is added and the mixture is seeded with clopidogrel hydrogen sulphate Form 2, the product is crystallized, the mixture is cooled, filtered and the crystals are washed and then dried under reduced pressure to give clopidogrel hydrogen sulphate Form 2.

12. Pharmaceutical composition containing, as active ingredient, the Form 2 polymorph of clopidogrel hydrogen sulphate according to Claim 1 in combination with at least one pharmaceutical excipient.
